# EUROPÄISCHE PATENTANMELDUNG

(11) **EP 2 465 558 A1**
(43) Veröffentlichungstag der Anmeldung: **20.06.2012**
(21) Anmeldenummer: 10195706.6
(22) Anmeldetag: 17.12.2010
(51) Int. Cl.: A61M 5/162

(54) **Vorrichtung für die Entnahme einer Flüssigkeit aus einem Behälter**

(71) Anmelder: Weibel CDS AG, 9104 Waldstatt (CH)
(72) Erfinder: Weibel, Ludwig Daniel, 9104, Waldstatt (CH)
(74) Vertreter: Hepp Wenger Ryffel AG

(57) **Zusammenfassung**

Auf einen mit einem Verschlusselement (4) verschlossenen und mit einer Flüssigkeit (2) gefüllten Behälter (3) ist eine Vorrichtung (1) aufgesetzt, mit deren Hilfe die Flüssigkeit entnommen werden kann. Diese Vorrichtung verfügt über ein Penetrationselement (6) mit einer Hohlnadel (9), das an einem Aufsatz (5) in einer Ruhelage (R) gehalten ist. Das Penetrationselement ist mit Führungsmitteln (7) am Aufsatz (5) gelagert, so dass es gegen das Verschlusselement (4) in eine Entnahmelage gepresst werden kann, in welcher die Hohlnadel (9) das Verschlusselement penetriert. Für den Druckausgleich im Behälter bei der Entnahme der Flüssigkeit ist wenigstens ein Belüftungskanal (10) vorgesehen, der vorzugsweise entlang der Hohlnadel (9) verläuft. Die aus der Atmosphäre angesaugte Luft gelangt über ein Filter (11) in den Belüftungskanal. Auf diese Weise wird eine Kontamination der Flüssigkeit verhindert und die Flüssigkeit kann ohne Gefahr einer Rückströmung entnommen werden.

## Beschreibung

Die Erfindung betrifft eine Vorrichtung für die Entnahme einer Flüssigkeit aus einem Behälter gemäss dem Oberbegriff von Anspruch 1. Dabei handelt es sich in der Regel um Vorrichtungen für die nadellose Entnahme von pharmazeutischen Substanzen aus einem hermetisch verschlossenen Glasgefäss. Die Flüssigkeit kann dabei in eine Injektionsspritze überführt werden, wobei die Nadel erst nachträglich aufgesetzt wird. Selbstverständlich können derartige Vorrichtungen aber auch im nichtmedizinischen Bereich eingesetzt werden, beispielsweise für chemische Analysezwecke im Lebensmittelbereich usw.

Durch die WO 01/60436 ist eine gattungsmässig vergleichbare Vorrichtung bekannt geworden, bei der eine linear verschiebbare Aufstechspitze in einem rohrförmigen Gehäuse gehalten ist, das auf die geschlossene Behältermündung aufgesetzt ist. Die Aufstechspitze wird in der oberen Ruhelage durch flexible Rastnocken fixiert, und sie weist eine umlaufende Verzahnung auf, welche in korrespondierende seitliche Zahnsegmente eingreifen. Bei einem Druck von oben in Richtung Behälter nach dem Anschluss einer Spritze löst sich die Aufstechspitze aus der Verrastung und gleitet in der Verzahnung drehfest nach unten, bis der Verschlussstopfen auf dem Behälter durchstochen ist. Anschliessend kann mit der Injektionsspritze der Behälterinhalt angesaugt werden.

Ein erheblicher Nachteil bekannter Vorrichtungen besteht darin, dass auch nach dem Durchstossen des Verschlusselements mit der Hohlnadel das Innere des Behälters gegenüber der Atmosphäre abgedichtet ist, wenn an das Penetrationselement ein anderer Behälter wie z.B. eine Injektionsspritze angeschlossen wird. Beim Ansaugen der Flüssigkeit aus dem Behälter entsteht somit ein Unterdruck, der im Extremfall dazu führt, dass der grösste Teil der Flüssigkeit wieder in den Behälter zurückströmt, sobald die Pumpbewegung unterbrochen wird. Aufgrund der Flexibilität des Verschlusselements kann zwar bei starkem Unterdruck im Behälter allenfalls Luft entlang der Aussenseite der Hohlnadel nachströmen. Dies ist allerdings nicht erwünscht, weil dadurch mikrobiologische Verunreinigungen in den Behälter und damit in die Flüssigkeit gelangen können.

Es ist daher eine Aufgabe der Erfindung, eine Vorrichtung der eingangs genannten Art zu schaffen, bei der die erwähnten Nachteile vermieden werden und bei der insbesondere unter Aufrechterhalten von sterilen Bedingungen ein Rückströmen von Flüssigkeit in den Behälter vermieden wird. Die Vorrichtung soll ausserdem zuverlässig funktionieren und auch von nicht besonders geschultem Personal leicht zu bedienen sein. Ausserdem soll sie einfach und preiswert herzustellen sein, da die Vorrichtung Bestandteil einer Einwegverpackung ist, die nach Gebrauch entsorgt werden muss.

Diese Aufgaben werden erfindungsgemäss mit einer Vorrichtung gelöst, welche die Merkmale im Anspruch 1 aufweist. Der wenigstens eine vorzugsweise entlang der Hohlnadel verlaufende Belüftungskanal bewirkt ersichtlicherweise, dass das Innere des Behälters in der Entnahmelage mit der Atmosphäre kurzgeschlossen wird. Der Aufbau eines stetig steigenden Unterdrucks im Behälter wird damit vermieden, da jeweils so viel Luft in den Behälter nachströmt, wie Flüssigkeit entfernt wird. Trotzdem ist eine schmutzfreie und sogar eine keimfreie Umgebung im Behälter gewährleistet, wenn gemäss einer weiteren Ausgestaltung die zugeführte Luft ein Filter passieren muss. Je nach Beschaffenheit dieses Filters können auch kleinste Partikel oder Lebewesen zurückgehalten werden. Selbstverständlich ist es denkbar, mehrere Lüftungskanäle anzuordnen und diese können entlang der Hohlnadel entweder linear oder auch schraubenlinienförmig verlaufen. Denkbar wäre auch die Anordnung eines Belüftungskanals an einem von der Hohlnadel getrennten Element, beispielsweise in der Form einer separaten Belüftungsnadel.

Besonders vorteilhaft ist jedoch der Belüftungskanal als Nut auf der Aussenseite der Hohlnadel ausgebildet. Vorzugsweise sind es mehrere parallele Nuten. Ein derartiges Bauteil ist einfach herzustellen und bei ausreichender Nuttiefe wird selbst dann ein genügend grosser Durchtrittsquerschnitt freigehalten, wenn das Verschlusselement aus sehr weichem und elastischem Material besteht.

Das Filter kann grundsätzlich am Penetrationselement angeordnet sein und sich zusammen mit diesem verschieben, oder es kann starr am Aufsatz gehalten sein. Wichtig ist in jedem Fall, dass die dem Belüftungskanal zugewandte Seite des Filters Bestandteil eines gegenüber der Atmosphäre steril abgegrenzten Raumes ist. Das Filter kann beispielsweise am Penetrationselement vorzugsweise am atmosphärenseitigen Ende des Belüftungskanals angeordnet sein, und zwar beispielsweise an einem die Hohlnadel umgebenden Kragen, der mit Öffnungen versehen ist und der gleichzeitig das Filter abstützt bzw. trägt. Diese nahe am Belüftungskanal angeordnete Variante hat den Vorteil, dass das Filter flächenmässig relativ klein gehalten werden kann.

Bei einer Zuordnung des Filters am Aufsatz erfolgt die Anordnung vorzugsweise in einem dem Verschlusselement zugewandten Bereich. Das Filter kann dabei in einer Halterung angeordnet sein, welche das Penetrationselement kreisringförmig umgibt. Diese Anordnung hat den Vorteil, dass der in der Ruhelage steril zu haltende Raum sehr klein gehalten werden kann.

Weitere Vorteile können erreicht werden, wenn die Führungsmittel wenigstens einen Wandabschnitt aufweisen, der das Penetrationselement mit dem Aufsatz verbindet und dieses in der Ruhelage hält, wobei der Wandabschnitt derart verformbar ist, dass er das Penetrationselement bis zum Erreichen der Entnahmelage führt. Durch die feste Verbindung zwischen Aufsatz und Penetrationselelement ist einerseits eine Verdrehsicherung gewährleistet, anderseits in Folge der Verformbarkeit aber auch eine gute Geradeführung. Eine derartige Ausgestaltung der Führungsmittel wäre auch an konventionellen Vorrichtungen ohne Entlüftungskanal und ohne Filter sehr vorteilhaft.

Der verformbare Wandabschnitt kann beispielsweise wenigstens eine das Penetrationselement umgebende, federelastische Membran sein. Diese kann derart verformbar sein, dass sie das Penetrationselement in der Entnahmelage unter der Federvorspannung fixiert. Die kuppelartig ausgebildete Membran ist somit in Folge ihrer Federeigenschaften sowohl in der Ruhelage als auch in der Entnahmelage vorgespannt. Dabei wird der "Knackfrosch"-Effekt der Membran ausgenutzt. Selbstverständlich können zum Fixieren des Penetrationselements in der Entnahmelage aber auch Rastmittel angeordnet sein, so dass die Federvorspannung der Membran für das Halten der Entnahmelage unerheblich ist.

Die Membran kann ganz unterschiedlich ausgebildet sein und beispielsweise auch in Umfangsrichtung aus mehreren, von einander abgetrennten Segmenten bestehen. Über die Zwischenräume zwischen den Segmenten kann in der Entnahmelage Atmosphärenluft nachströmen. Es ist aber auch denkbar, dass eine in der Ruhelage geschlossene Membran mit Sollbruchstellen versehen ist, welche beim Überführen des Penetrationselements in die Entnahmelage aufplatzen. Derartige Sollbruchstellen haben den Vorteil, dass die Membran in der Ruhelage relativ formsteif ist und erst nach dem Aufplatzen der Sollreisslinien eine erhöhte Elastizität aufweist. Ausserdem kann auch hier in der Entnahmelage über die aufgeplatzten Sollreisslinien Luft aus der Atmosphäre nachströmen.

Alternativ kann das Führungsmittel aber auch wenigstens zwei Stege aufweisen, welche die genannten Wandabschnitte bilden und von denen jeder derart mit wenigstens einem Biegegelenk versehen ist, dass die Stege bei einer Verschiebung des Penetrationselements in die Entnahmelage zusammenfaltbar sind. Die Stege bilden auf diese Weise spinnenartige Beine des Penetrationselements, die sich im Verlauf der Linearbewegung vorzugsweise nach aussen falten lassen.

Alternative Ausgestaltungen der Führungsmittel wären selbstverständlich denkbar. So könnte der Wandabschnitt beispielsweise auch als Faltenbalg ausgebildet werden, der sich zieharmonikaartig zusammenpressen lässt. Denkbar wäre auch eine Anzahl miteinander verbundene Ringe oder Ringsegmente, die sich teleskopartig zusammenschieben lassen. Ein wesentlicher Vorteil wird in jedem Fall erreicht, wenn die Führungsmittel einstückig mit dem Aufsatz und/oder mit dem Penetrationselement verbunden sind. Die gesamte Struktur lässt sich auf diese Weise als Spritzgussteil in Kunststoffmaterial einstückig herstellen. In bestimmten Fällen kann es jedoch fabrikationstechnisch auch zweckmässig sein, einzelne Bauteile separat herzustellen und anschliessend miteinander zu verschweissen oder auf andere Weise miteinander zu verbinden. Auf diese Weise könnten unterschiedliche Materialeigenschaften optimal miteinander gepaart werden. Optimal sind dabei Werkstoffe, welche mit verschiedenen Verfahren sterilisierbar sind (z. B. strahlensterilisierbar, dampfsterilisierbar), ohne dabei Schaden zu erleiden.

Um einen keimfreien und möglichst luft- bzw. flüssigkeitsdichten Anschluss an das Penetrationselement zu gewährleisten, ist dieses mit einem entsprechenden Kupplungsteil versehen. Dabei kann es sich beispielsweise um den Innenkegel eines Luer-Locks handeln. Aber auch andersartige Kupplungsmittel wie z.B. beliebige Schnappverschlüsse oder dergleichen sind denkbar.

Das Penetrationselement kann ausserdem Rastmittel aufweisen, mit denen es in der Entnahmelage verrastbar ist. Dadurch ist gewährleistet, dass bei der Entnahme der Flüssigkeit keine unerwünschten Relativbewegungen auftreten.

Der Aufsatz kann einen Zentrierring tragen, der die Hohlnadel zusätzlich führt und zentriert. Das Durchstechen des Verschlusselements erfolgt so an einer genau definierten Stelle. Ausserdem werden unerwünschte Querbewegungen durch fehlerhafte Handhabung beim Einstechen vermieden.

Zum Schutz des Penetrationselements in der Ruhelage und auch als Garantiesicherung ist das Penetrationselement durch eine lösbare Schutzkappe abgedeckt. Diese Schutzkappe muss ausreichend Stabilität aufweisen, um auch bei starken Schlägen und Stössen eine Verschiebung des Penetrationselements zu verhindern. Als Garantiesicherung kann die Schutzkappe beispielsweise ein abtrennbares Garantieband aufweisen. Die Schutzkappe hat zusätzlich auch noch die Aufgabe die Vorrichtung keimfrei abzudichten. Dies setzt voraus, dass die Schutzkappe dichtend gegen das Verschlusselement und/oder gegen die Behältermündung gepresst wird. Dies kann beispielsweise über eine Manschette erfolgen, welche um den Aufsatz und um die Behältermündung aufgeschrumpft oder angerollt oder angebördelt wird. Bei einer Ausbildung dieser Manschette beispielsweise aus Aluminium oder aus einer Aluminiumlegierung kann das eine Ende der Manschette bei der Herstellung direkt in den Aufsatz eingegossen werden. Nach dem Füllen und Verschliessen des Behälters kann das freie Ende um die Behältermündung angerollt oder angebördelt werden.

Die Schutzkappe kann besonders vorteilhaft auch mit einem Gewinde versehen und auf den Aufsatz und/oder auf das Penetrationselement aufgeschraubt sein. Eine Fixierung der Schraubkappe könnte beispielsweise über eine Schweissung erfolgen.

Eine sehr vorteilhafte Möglichkeit der Befestigung auf einer Behältermündung besteht auch darin, dass der Aufsatz auf der Behältermündung aufgeschnappt ist. Bei entsprechend ausgebildeten Rastmitteln kann dabei eine relativ hohe Anpresskraft aufgebaut werden. Vergleichbare Schnappverbindungen auf Behältermündungen sind beispielsweise bereits bei Verschlüssen in der Lebensmittelindustrie bekannt.

Eine weitere sehr vorteilhafte Ausführung besteht in der Wahl von möglichst transparenten Kunststoffen für die einzelnen Bauteile. Dadurch kann das fertig abgefüllte und montierte Produkt zerstörungsfrei visuell auf jegliche Defekte inspiziert werden.

Weitere Vorteile und Einzelmerkmale der Erfindung sind in den Zeichnungen dargestellt und werden nachstehend beschrieben. Es zeigen:
- Figur 1:: Einen stark vergrösserten Querschnitt durch eine erfindungsgemässe Vorrichtung in Ruhelage,
- Figur 2:: die Vorrichtung gemäss Figur 1 in Entnahmelage mit angesetzter Spritze,
- Figur 3:: einen Schnitt durch die Ebene I-I gemäss Figur 1,
- Figur 4:: einen Schnitt durch die Ebene II-II gemäss Figur 1,
- Figur 5:: einen Querschnitt durch ein alternatives Ausführungsbeispiel einer Vorrichtung,
- Figur 6:: die Vorrichtung gemäss Figur 5 in Entnahmelage,
- Figur 7:: ein weiteres leicht abgewandeltes Ausführungsbeispiel der Vorrichtung gemäss Figur 5,
- Figur 8:: die Vorrichtung gemäss Figur 7 in Entnahmelage,
- Figur 9:: einen Querschnitt durch eine erfindungsgemässe Vorrichtung mit integriertem Entnahmeventil, und
- Figur 10:: einen Querschnitt durch ein weiteres Ausführungsbeispiel einer Vorrichtung.

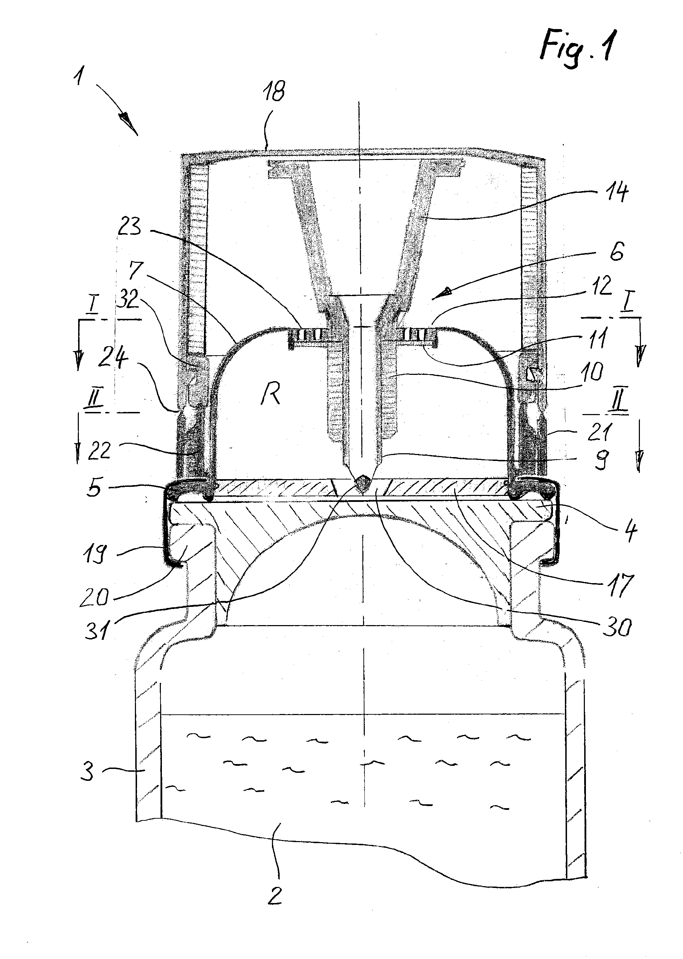

Figur 1 zeigt eine Anordnung bestehend aus einer erfindungsgemässen Vorrichtung 1, welche auf die Behältermündung 20 eines Behälters 3 aufgesetzt ist. Der Behälter 3, beispielsweise aus Glas gefertigt, ist mit einer Flüssigkeit 2 gefüllt und mit einem Verschlusselement 4, beispielsweise aus Gummi luftdicht verschlossen. Bei der Flüssigkeit 2 kann es sich um einen pharmazeutischen Wirkstoff handeln, der dazu bestimmt ist, mit einer Injektionsspritze entweder direkt in den menschlichen oder tierischen Körper injiziert zu werden oder aber einem Infusionssystem beigemischt zu werden.

Die Vorrichtung 1 besteht im Wesentlichen aus einem Aufsatz 5 aus Kunststoffmaterial, der mit seiner Unterseite dichtend auf dem äusseren Randbereich des Verschlusselements 4 aufliegt und der über eine kuppelartig gewölbte Membran 7 mit einem zentralen Penetrationselement 6 verbunden ist. Der Aufsatz 5 ist einstückig mit einem Wandabschnitt 22 verbunden, der als Widerlager für ein aussen liegendes Garantieband 21 dient. Die dichtende und formschlüssige Verbindung des Aufsatzes 5 mit der Behältermündung 20 erfolgt über eine Manschette 19 aus Aluminium, welche direkt in den Aufsatz 5 eingegossen ist und welche an ihrem freien Ende an die Behältermündung 20 angerollt ist. In einer Ebene unmittelbar über dem Verschlusselement 4 trägt der Aufsatz einen Zentrierring 17 mit einer zentralen Öffnung 30, der beispielsweise eingeschnappt werden kann.

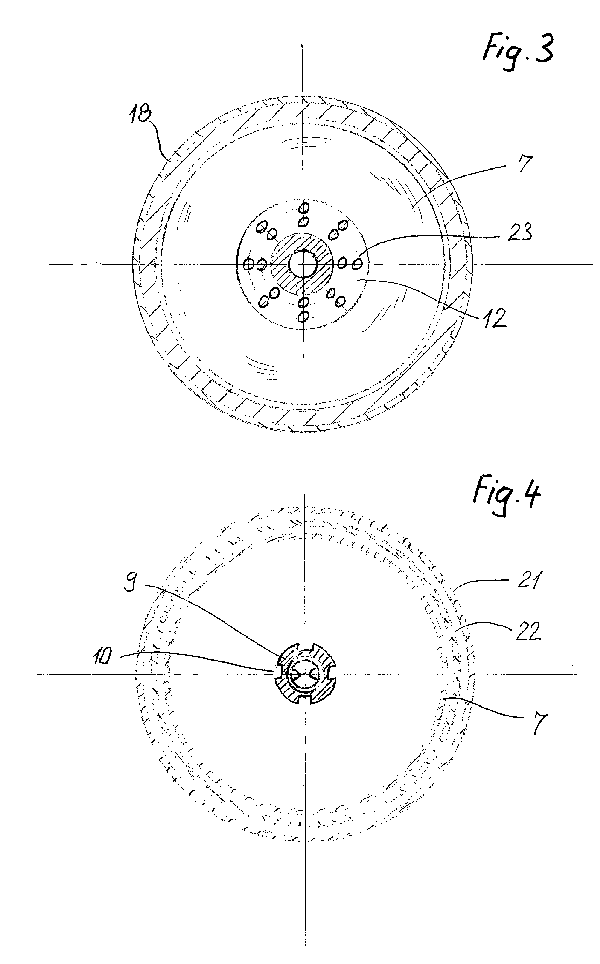

Das vorzugsweise rotationssymmetrisch ausgebildete Penetrationselement 6 verfügt im mittleren Abschnitt über einen umlaufenden Kragen 12, in welchem Ansaugöffnungen 23 angeordnet sind (Figur 3). Unterhalb des Kragens ist ein mikrobiologisch dichtes Filter 11 angeordnet, das sämtliche Ansaugöffnungen 23 überdeckt. Über dem Kragen 12 ist integral der Innenkonus eines Luer-Locks 14 angeordnet. Dieser geht unmittelbar in eine Hohlnadel 9 über, welche sich unterhalb des Kragens 12 nach unten erstreckt und welche in eine mit Öffnungen versehene Aufstechspitze 31 mündet. Der Begriff Hohlnadel wird hier für jedes Element verwendet, das einen Ansaugkanal bildet und das in der Lage ist, das Verschlusselement 4 zu penetrieren.

Wie insbesondere auch aus Figur 4 ersichtlich ist, hat die Hohlnadel 9 eine relativ dicke Wandung, entlang welcher vorzugsweise parallele Belüftungskanäle 10 in der Form von Nuten angeordnet sind. Diese Nuten erstrecken sich bis unmittelbar unter den Kragen 12. Das gesamte Penetrationselement 6 ist abgesehen vom Filter 11 vorzugsweise einstückig ausgebildet. Es kann in die Membran 7 eingeschweisst oder eingeschnappt sein oder es kann gar mit dieser ebenfalls einstückig ausgebildet sein. Die Belüftungskanäle können im Querschnitt auch als Schwalbenschwanznut ausgebildet sein. Damit kann die Gefahr reduziert werden, dass das elastische Material des Verschlusselements 4 die Belüftungskanäle verschliesst.

Die Membran 7 ist federelastisch ausgebildet, so dass sie in der dargestellten Ruhelage R das Penetrationselement 6 nach oben vorspannt. In dieser Ruhelage ist das Penetrationselement von einer Schutzkappe 18 abgedeckt, welche über eine Sollbruchlinie 24 mit dem Garantieband 21 verbunden ist. Ein Dichtring 32 zwischen dem Wandabschnitt 22 und der Schutzkappe 18 sorgt dafür, dass der gesamte Innenraum der Schutzkappe über dem Verschlusselement 4 steril und hermetisch abgedichtet ist. Die Schutzkappe 18 kann aus Kunststoffmaterial oder auch aus Aluminium gefertigt sein. In bestimmten Fällen wäre es denkbar, dass der Luer-Lock 14 in der Ruhelage R durch die Schutzkappe 18 gehalten bzw. zentriert wird.

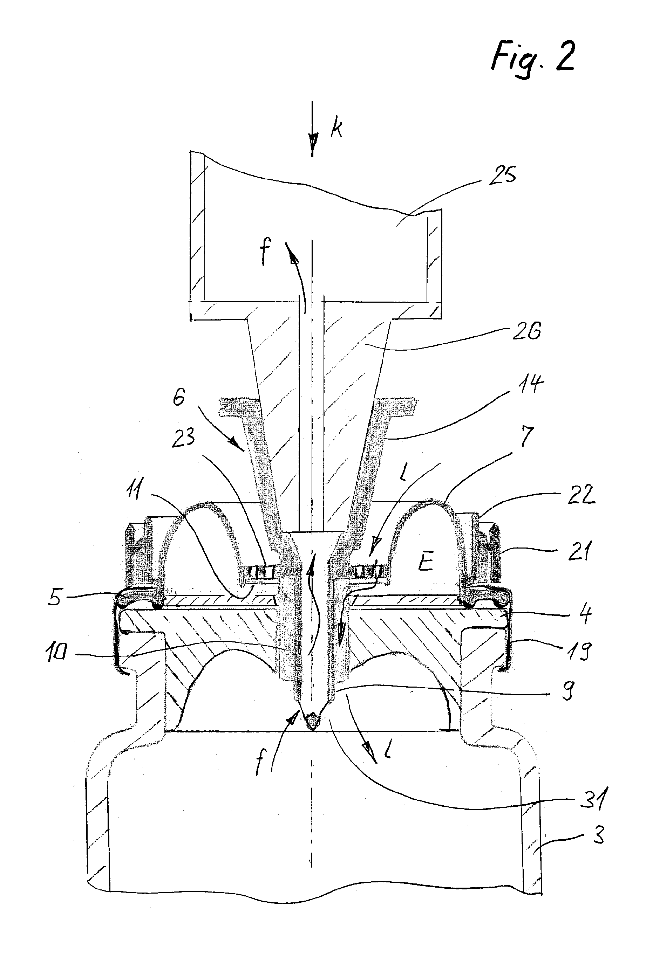

Anhand von Figur 2 wird nachstehend die Entnahme der Flüssigkeit aus dem Behälter 3 in der Entnahmelage E erläutert. Bevor die Entnahmelage erreicht wird, muss zuerst die Schutzkappe 18 entfernt werden, so dass am Aufsatz 5 nur noch das Garantieband 21 zurückbleibt. Je nach Garantiesystem könnte beim Öffnen der Schutzkappe auch das Garantieband wegfallen. Sofort nach dem Entfernen der Schutzkappe wird der Gegenkonus 26 einer Spritze 25 in den Luer-Lock 14 eingeführt und es wird eine Kraft in Pfeilrichtung k auf das Penetrationselement 6 ausgeübt. Dabei durchdringt die Hohlnadel 9 das Verschlusselement 4, so dass die Aufstechspitze 31 ins Innere des Behälters 3 hineinragt. Die Belüftungskanäle 10 schaffen dabei zusätzlich eine Verbindung zwischen dem Innenraum des Behälters 3 und der Innenseite der Membran 7.

In der Entnahmelage E wird die Membran 7 derart stark in Richtung Verschlusselement gekrümmt, dass die Federvorspannung die Kraftrichtung ändert und das Penetrationselement in der Entnahmelage fixiert. Die Reibung zwischen der Hohlnadel 9 und dem Verschlusselement 4 trägt weiter dazu bei, dass das Penetrationselement in dieser Position verbleibt.

Die eigentliche Entnahme der Flüssigkeit durch Aufziehen der Spritze 25 erfolgt selbstverständlich nicht in der dargestellten Position, sondern kopfüber, so dass die Flüssigkeit die Innenseite des Verschlusselements 4 flutet. Durch Aufziehen der Spritze fliesst so die Flüssigkeit in Pfeilrichtung f durch die Hohlnadel 9 in die Spritze 25. Der dabei entstehende Unterdruck über dem Flüssigkeitsspiegel wird ausgeglichen durch aus der Atmosphäre angesaugte Luft, welche in Pfeilrichtung 1 über die Ansaugöffnungen 23, durch das Filter 11 und über die Belüftungskanäle 10 in den Behälter 3 gelangt. Eine Kontamination der Flüssigkeit ist dabei nicht möglich, weil Schmutzteile oder beispielsweise auch Bakterien am Filter 11 ausgefiltert werden.

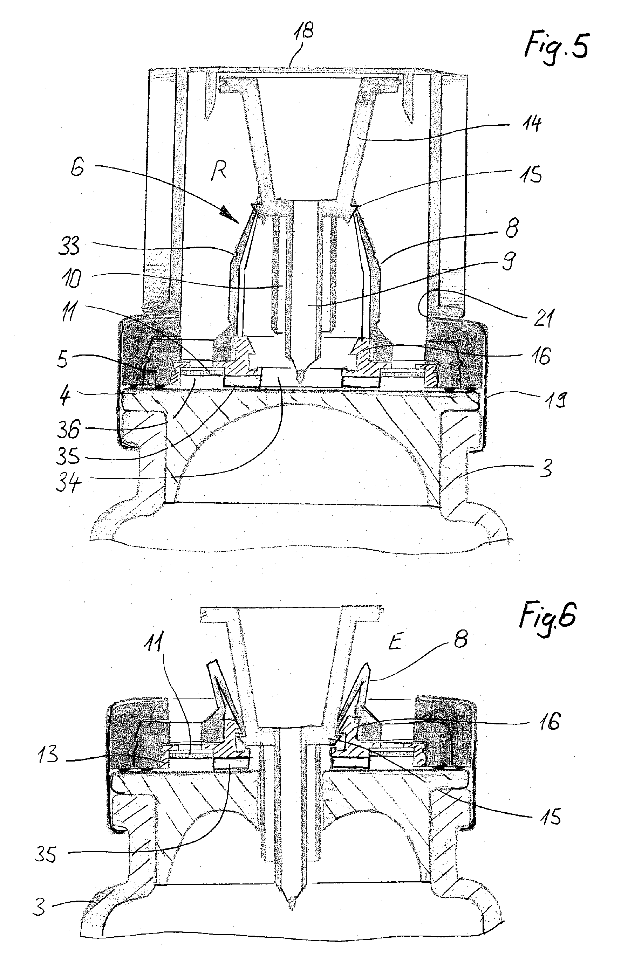

In den Figuren 5 und 6 ist ein alternatives Ausführungsbeispiel einer Vorrichtung dargestellt, wobei gleiche Bauteile mit den gleichen Bezugszeichen versehen sind, wie beim Ausführungsbeispiel gemäss den Figuren 1 und 2. Der wesentliche Unterschied besteht einerseits in der Verbindung zwischen dem Aufsatz 5 und dem Penetrationselement 6 und anderseits in der Anordnung des Filters 11. Auch die Verschlusskappe 18 ist etwas anders aufgebaut und nicht mit einem separaten Garantieband versehen. Vielmehr befindet sich die Sollbruchlinie 24 unmittelbar über dem eingegossenen oberen Rand der Manschette 19.

Der Aufsatz 5 ist mit dem Penetrationselement 6 über vier Materialstege 8 verbunden, welche um die Hohlnadel 9 gleichmässig verteilt sind. Jeder Materialsteg verfügt über wenigstens ein Biegegelenk 33. Die Materialstege 8 bilden auf diese Weise spinnenbeinartige Stützen, welche das Penetrationselement 6 in der Ruhelage halten. Der Luer-Lock 14 ist am Boden der Verschlusskappe 18 zusätzlich fixiert.

Beim vorliegenden Ausführungsbeispiel ist das Filter 11 nicht am Penetrationselement, sondern am Aufsatz 5 fixiert. Dazu ist eine separate Filterhalterung 13 vorgesehen, welche eine zentrale Öffnung aufweist und welche die Hohlnadel 9 kreisringförmig umgibt. Die innere, nabenartige Partie der Filterhalterung liegt auf dem Verschlusselement 4 auf, wobei jedoch die zentrale Öffnung 34 über Nuten 35 mit dem kreisringförmigen Hohlraum 36 verbunden ist, in dem das Filter 11 fixiert ist. An der Filterhalterung 13 ist ein nach innen ragender Rastring 16 angeordnet. Ebenso ist am Penetrationselement 6 ein nach aussen ragender Rastring 15 angeordnet.

In der Entnahmelage gemäss Figur 6 sind die Materialstege 8 um die Biegegelenke 33 gefaltet und ragen dabei etwas nach aussen. Die Rastringe oder Rastnasen 15 und 16 sind miteinander verrastet und fixieren auf diese Weise das Penetrationselement, dessen Hohlnadel 9 das Verschlusselement 4 durchstossen hat. Luft aus der Atmosphäre gelangt durch das Filter 11 über die Nuten 35 und die Belüftungskanäle 10 ins Innere des Behälters 3.

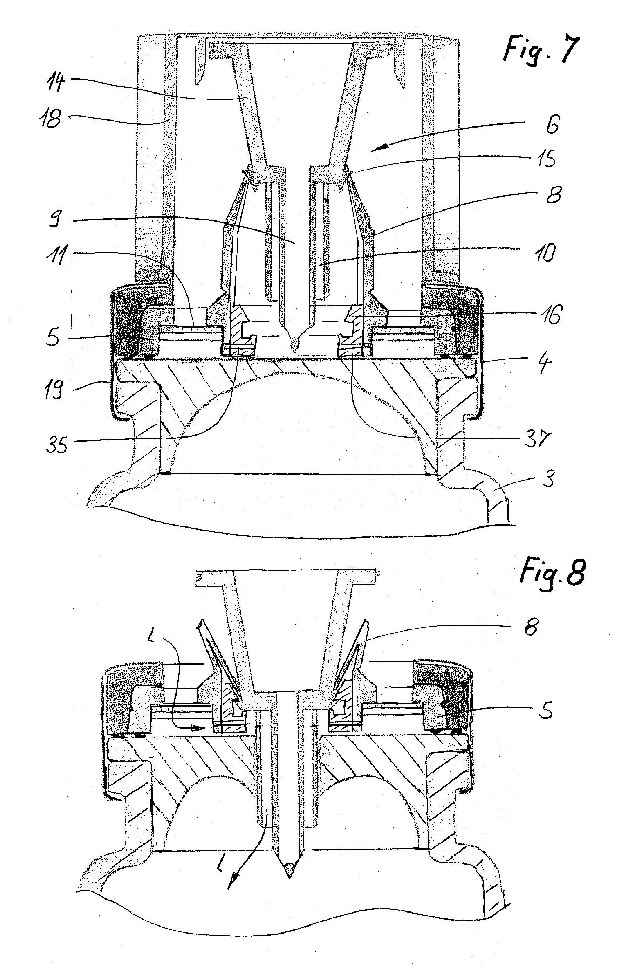

Das Ausführungsbeispiel gemäss den Figuren 7 und 8 unterscheidet sich von demjenigen gemäss den Figuren 5 und 6 nur durch die Art und Weise, wie das Filter 11 fixiert ist. Die Fixierung des Filters erfolgt hier nicht durch eine separate Filterhalterung, sondern unmittelbar durch einen Abschnitt des Aufsatzes 5. Separat ausgebildet ist hier lediglich ein Innenring 37, der den nach innen ragenden Rastring 16 trägt.

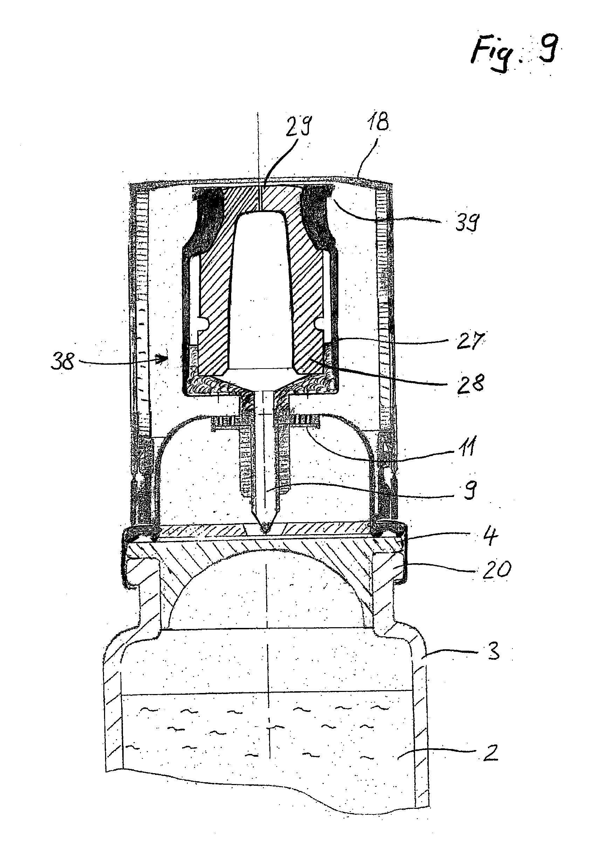

Das Ausführungsbeispiel gemäss Figur 9 entspricht weitgehend demjenigen gemäss Figur 1. In dem mit einem Ventilgehäuse 27 ausgebildeten, speziellen Kupplungsteil 39 ist ein mit 38 bezeichnetes Ventil eingebaut. Dieses Ventil ermöglicht es, in der Entnahmelage in mehreren zeitlich voneinander getrennten Sequenzen Flüssigkeit aus dem Behälter 3 zu entnehmen, wobei nach jeder Entnahme eine sterile Abdichtung gewährleistet ist. Im Ventilgehäuse 27 ist ein Ventilkörper 28 aus einem weichelastischen Material gelagert. In diesem Ventilkörper ist ein Schlitz 29 angeordnet, der im Ruhezustand geschlossen ist, so dass der Ventilkörper 28 wie eine Dichtung wirkt. Beim Ansetzen einer Spritze in den Luer-Lock 39 wird der Schlitz 29 aufgespreizt und gibt so den Zugang zur Hohlnadel 9 frei. Ein derartiges Ventil ist beispielsweise beschrieben in der WO 03/064907. Bedingt durch die grössere Bauhöhe des Penetrationselements 6 muss ersichtlicherweise auch die Verschlusskappe 18 länger ausgebildet werden.

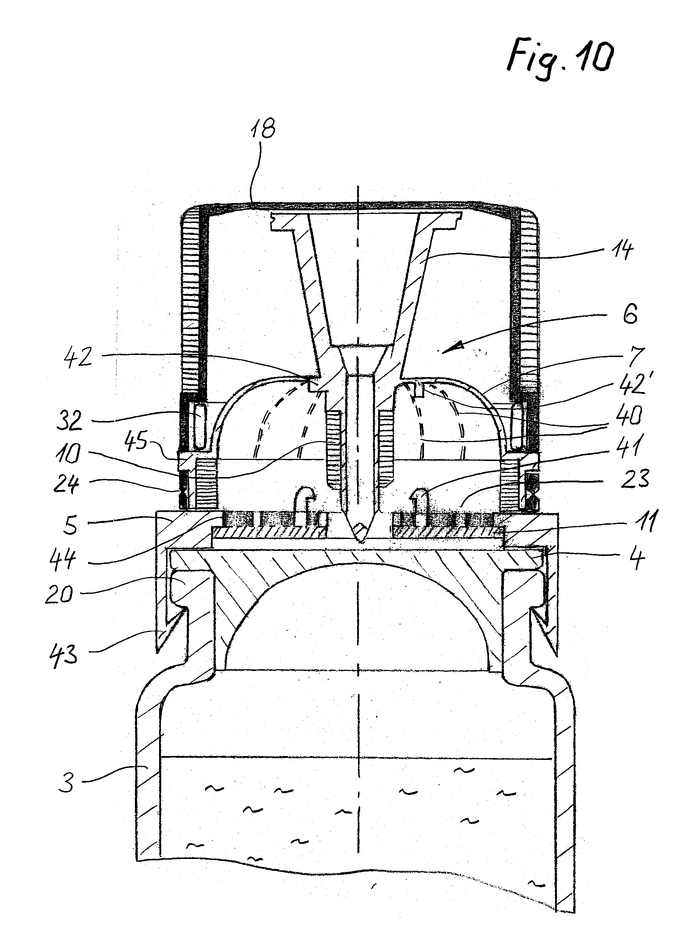

Das Ausführungsbeispiel gemäss Figur 10 hat zwar eine gewisse Ähnlichkeit mit demjenigen gemäss Figur 1, unterscheidet sich jedoch von diesem durch die Ausbildung der Membran 7, durch die Anordnung des Filters 11, sowie durch die Art der Befestigung auf der Behältermündung 20. Ausserdem wird das Penetrationselement 6 in der Entnahmelage durch eine Rastverbindung fixiert.

Die domartig gewölbte Membran 7 ist über den Umfang verteilt mit Sollbruchstellen oder Sollbruchlinien 40 versehen, welche sich etwa parallel zur Längsmittelachse vom Zenit der Kuppel bis zu deren unteren Rand erstrecken. Diese Sollbruchstellen können dabei als Schwächungslinien ausgebildet sein, oder es kann sich auch um Perforationen handeln. Beim Überführen des Penetrationselements aus der dargestellten Ruhelage in die Entnahmelage (analog zu Figur 2) platzen die Sollbruchstellen 40 auf, wobei schlitzartige Öffnungen entstehen. In der Entnahmelage rasten am Penetrationselement angeordnete Rastblöcke 42 in die Rasthaken 41 am Aufsatz 5 und fixieren auf diese Weise die Entnahmelage. Die Rastblöcke können ganz unterschiedlich ausgestaltet sein und beispielsweise wie in der linken Bildhälfte dargestellt, starr am Penetrationselement angeordnet sein. Denkbar ist aber auch eine Anordnung an der Membran 7, wie in der rechten Bildhälfte dargestellt (42'), wobei jeder Rastblock bis zum Erreichen der korrespondierenden Rasthaken 41 eine Schwenkbewegung von ca. 90° ausführt.

Der Aufsatz 5 ist hier nicht mittels einer Manschette an der Behältermündung fixiert, sondern mit Hilfe eines Schnappkragens 43 auf diese aufgeschnappt. Dieser Schnappkragen ist derart dimensioniert, dass einerseits eine dichtende Presskraft auf das Verschlusselement 4 ausgeübt wird und dass anderseits eine Entfernung der Schutzkappe 18 möglich ist, ohne dass unbeabsichtigt der Aufsatz 5 vom Behälter 3 abgehoben wird.

Das Filter 11 ist hier dem Aufsatz 5 zugeordnet, wobei es direkt unterhalb einer mit Ansaugöffnungen 23 versehenen Trägerplatte 44 befestigt ist. Die nachströmende Luft fliesst auf diese Weise über die geöffneten Sollbruchstellen 40, durch die Ansaugöffnungen 23 und das Filter 11 und danach über die Belüftungskanäle 10 ins innere des Behälters 3.

Beim vorliegenden Ausführungsbeispiel ist auch die Schutzkappe 18 mit der Sollbruchlinie 24 etwas abgewandelt ausgestaltet. Die domartige Membran 7 geht an ihrem unteren Rand in einen umlaufenden Stützring 45 über, der beispielsweise durch eine Schweissung einstückig mit dem Aufsatz 5 verbunden ist. Auf diesem Stützring ist eine Dichtung 32 abgestützt, welche die Innenseite der Schutzkappe 18 nach aussen hermetisch abdichtet. Der Dichtring 32 könnte auch als Zweikomponenten-Spritzgussteil direkt in die Schutzkappe 18 integriert sein. Der Materialstreifen unterhalb der Sollbruchlinie 24 ist als Garantieband ausgestaltet, nach dessen Entfernung die Schutzkappe 18 vom Stützring 45 abgehoben werden kann.

Für sämtliche erfindungsgemässen Ausführungen können als Filter neben den gängigen Mikrofiltermembranen vorteilhaft Polykarbonat-Folien verwendet werden, welche von feinsten Öffnungen durchsetzt sind. Anstelle von separat eingelegten oder angeklebten Filtern könnten diese auch direkt beim Spritzgiessen der jeweiligen Tragkonstruktionen integriert werden. Ein wesentlicher Vorteil sämtlicher erfindungsgemässen Varianten besteht auch noch darin, dass diese für Behälter mit einem Bördelrand von 13 mm Durchmesser verwendet werden können.

## Patentansprüche

1. Vorrichtung (1) für die Entnahme einer Flüssigkeit (2) aus einem Behälter (3), der mit einem penetrierbaren Verschlusselement (4) verschlossen ist, mit einem dichtend an das Verschlusselement anschliessbaren Aufsatz (5), in welchem ein Penetrationselement (6) derart gelagert ist, dass es mit Hilfe von Führungsmitteln (7, 8) zwischen einer Ruhelage (R) und einer Entnahmelage (E) verschiebbar ist, wobei das Penetrationselement wenigstens eine Hohlnadel (9) aufweist, mit welcher in der Entnahmelage das Verschlusselement penetrierbar ist, **gekennzeichnet durch** wenigstens einen vorzugsweise entlang der Hohlnadel verlaufenden Belüftungskanal (10) zum Belüften des Behälters (3) bei der Entnahme der Flüssigkeit.

2. Vorrichtung nach Anspruch 1, **dadurch gekennzeichnet, dass** sie wenigstens ein Filter (11) aufweist, über das in der Entnahmelage Luft aus der Atmosphäre über den Belüftungskanal (10) in den Behälter zuführbar ist.

3. Vorrichtung nach Anspruch 1 oder 2, **dadurch gekennzeichnet, dass** der Belüftungskanal als Nut auf der Aussenseite der Hohlnadel ausgebildet ist.

4. Vorrichtung nach Anspruch 2, **dadurch gekennzeichnet, dass** das Filter am Penetrationselement (6), vorzugsweise am atmosphärenseitigen Ende des Belüftungskanals angeordnet ist.

5. Vorrichtung nach Anspruch 4, **dadurch gekennzeichnet, dass** am atmosphärenseitigen Ende des Belüftungskanals (10) ein die Hohlnadel (9) umgebender Kragen (12) angeordnet ist und dass das Filter am Kragen gehalten ist.

6. Vorrichtung nach Anspruch 2, **dadurch gekennzeichnet, dass** das Filter (11) am Aufsatz (5), vorzugsweise in einem dem Verschlusselement zugewandten Bereich angeordnet ist.

7. Vorrichtung nach Anspruch 6, **dadurch gekennzeichnet, dass** das Filter (11) in einer Halterung (13) angeordnet ist, welche das Penetrationselement (6) kreisringförmig umgibt.

8. Vorrichtung nach einem der Ansprüche 1 bis 7, **dadurch gekennzeichnet, dass** die Führungsmittel (7, 8) wenigstens einen Wandabschnitt aufweisen, der das Penetrationselement (6) mit dem Aufsatz (5) verbindet und dieses in der Ruhelage (R) hält, wobei der Wandabschnitt derart verformbar ist, dass er das Penetrationselement bis zum Erreichen der Entnahmelage (E) führt.

9. Vorrichtung nach Anspruch 8, **dadurch gekennzeichnet, dass** der Wandabschnitt wenigstens eine das Penetrationselement umgebende, federelastische Membran (7) ist.

10. Vorrichtung nach Anspruch 8, **dadurch gekennzeichnet, dass** das Führungsmittel wenigstens zwei Stege (8) aufweist, von denen jeder derart mit wenigstens einem Biegegelenk (33) versehen ist, dass die Stege bei einer Verschiebung des Penetrationselements (6) in die Entnahmelage zusammenfaltbar sind.

11. Vorrichtung nach einem der Ansprüche 8 bis 10, **dadurch gekennzeichnet, dass** die Führungsmittel (7, 8) einstückig mit dem Aufsatz (5) und/oder mit dem Penetrationselement (6) verbunden sind.

12. Vorrichtung nach einem der Ansprüche 1 bis 11, **dadurch gekennzeichnet, dass** das Penetrationselement (6) ein Kupplungsteil, insbesondere den Innenkegel eines Luer-Locks aufweist.

13. Vorrichtung nach einem der Ansprüche 1 bis 12, **dadurch gekennzeichnet, dass** das Penetrationselement (6) Rastmittel aufweist, mit denen es in der Entnahmelage verrastbar ist.

14. Vorrichtung nach einem der Ansprüche 1 bis 13, **dadurch gekennzeichnet, dass** der Aufsatz (5) einen Zentrierring (17) zum Zentrieren der Hohlnadel trägt.

15. Vorrichtung nach einem der Ansprüche 1 bis 14, **dadurch gekennzeichnet, dass** das Penetrationselement (6) in der Ruhelage durch eine lösbare Schutzkappe (18) abgedeckt ist.

16. Vorrichtung nach Anspruch 15, **dadurch gekennzeichnet, dass** die Schutzkappe über eine Sollbruchstelle direkt oder indirekt mit dem Aufsatz (5) verbunden ist.

17. Vorrichtung nach einem der Ansprüche 1 bis 16, **dadurch gekennzeichnet, dass** am Penetrationselement (6) für die mehrfache Entnahme von Flüssigkeit in der Entnahmelage ein Ventil zum vorzugsweise sterilen Verschliessen der Hohlnadel angeordnet ist.

18. Anordnung mit einem eine Flüssigkeit enthaltenden Behälter, dessen Behältermündung mit einem penetrierbaren Verschlusselement (4) verschlossen ist, sowie mit einer Vorrichtung nach einem der Ansprüche 1 bis 17, wobei der Aufsatz (5) dichtend auf dem Verschlusselement aufliegt.

19. Anordnung nach Anspruch 18, **dadurch gekennzeichnet, dass** der Aufsatz auf der Behältermündung (20) aufgeschnappt ist.
